(19) Europäisches Patentamt
European Patent Office
Office européen des brevets

(11) **EP 3 239 700 B1**

(12) **EUROPÄISCHE PATENTSCHRIFT**

(45) Veröffentlichungstag und Bekanntmachung des Hinweises auf die Patenterteilung:
**27.09.2023  Patentblatt 2023/39**

(21) Anmeldenummer: **17167096.1**

(22) Anmeldetag: **19.04.2017**

(51) Internationale Patentklassifikation (IPC):
**G01N 23/04** (2018.01)      **A61B 6/00** (2006.01)

(52) Gemeinsame Patentklassifikation (CPC):
**A61B 6/4452; A61B 6/547; A61B 6/587; A61B 6/588; G01N 23/04;** G01N 2223/34

(54) **VERFAHREN ZUM BETREIBEN EINES RÖNTGENGERÄTES SOWIE RÖNTGENGERÄT**

METHOD FOR OPERATING AN X-RAY DEVICE AND X-RAY DEVICE

PROCÉDÉ DE FONCTIONNEMENT D'UN APPAREIL À RAYONS X ET APPAREIL À RAYONS X

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.04.2016  DE 102016207021**

(43) Veröffentlichungstag der Anmeldung:
**01.11.2017  Patentblatt 2017/44**

(73) Patentinhaber: **Siemens Healthcare GmbH 91052 Erlangen (DE)**

(72) Erfinder:
• **MÜLLER, Dominikus Joachim 82223 Eichenau (DE)**
• **SCHWARZER, Stefan 82024 Taufkirchen (DE)**
• **DORNBERGER, Susanne 91052 Erlangen (DE)**
• **SCHMIDT, Verena 92681 Erbendorf (DE)**

(56) Entgegenhaltungen:
**WO-A1-2012/127117     DE-A1-102010 020 782 US-A- 5 463 669**

EP 3 239 700 B1

**Beschreibung**

Die vorliegende Erfindung betrifft ein Verfahren zum Betreiben eines Röntgengerätes gemäß dem Oberbegriff des Patentanspruchs 1 sowie ein Röntgengerät gemäß dem Oberbegriff des Anspruchs 8.

Es ist bekannt, dass eine Kenntnis der relativen Lage und Position zwischen Strahler und Detektor eines Röntgengerätes notwendig ist, um ein definiertes Röntgenbild mit dem Röntgengerät, d.h. mit der Anordnung bestehend aus einem Röntgenstrahler und einem Röntgendetektor, zu erhalten.

Lage im Sinne des vorliegenden Dokumentes umfasst eine translatorische Verschiebung des Detektors relativ zum Strahler ebenso wie eine Verkippung/Verdrehung des Detektors relativ zum Strahler, jeweils in allen 3 Raumachsen, welche im Dokument im deutschen Sprachraum mit Roll-Nick-Gier-Winkel und im englischen Sprachraum, sowie dem vorliegenden Dokument mit roll-pitch-yaw Winkel bezeichnet werden können, die eine in der Fachwelt bekannte Möglichkeit zur Beschreibung der Orientierung eines Objekts im dreidimensionalen Raum darstellen.

Die Information bzgl. der relativen Lage und Position eines Röntgendetektors zum dazugehörigen Röntgenstrahler steht bei heutigen Geräten nicht zur Verfügung.

Aus der Druckschrift WO 2012/127117 A1 ist eine intra-orale Röntgenbildgebungsanordnung bekannt, die einen Bildgebungssensor, der im Mund eines Patienten positionierbar ist, und ein Steuersystem der Bildgebungsanordnung umfasst. Das Steuersystem ist in funktioneller Verbindung mit einem Mittel angeordnet, das so eingerichtet ist, dass es Messsignale an das Steuersystem sendet, die über die Position von mindestens einer in der Anordnung enthaltenen Komponente informieren. In Verbindung mit dem Abbildungssensor und einer in der Anordnung enthaltenen Strahlungsquelle sind Mittel angeordnet, um ihre räumliche Position zu messen und auf der Grundlage dieser Messungen Messsignale an das Steuersystem zu senden, das mit einem Mittel versehen ist, um auf der Grundlage der Messsignale Informationen über die gegenseitige räumliche Position des Abbildungssensors und der Strahlungsquelle oder einer an der Strahlungsquelle angeordneten Komponente zu bestimmen. Es ist Aufgabe der vorliegenden Erfindung, eine Lösung anzugeben, die eine verlässliche Bestimmung der Lage ermöglicht.

Diese Aufgabe wird durch ein Verfahren zum Betreiben eines Röntgengerätes gemäß den Merkmalen des Patentanspruchs 1 sowie durch das Röntgengerät gemäß den Merkmalen des Anspruchs 8 gelöst. Weitere vorteilhafte Weiterbildungen sind durch die Unteransprüche gegeben.

Beim erfindungsgemäßen Verfahren zum Betreiben eines aus einem Röntgenstrahler und Röntgendetektor bestehenden Röntgengerätes wird

a) am Röntgenstrahler ein magnetisches Wechselfeld erzeugt und ausgesandt,
b) am Röntgendetektor durch mindestens zwei zur Erfassung von jeweils zumindest einer mit dem magnetischen Wechselfeld korrelierenden der Magnetfeldstärke angebrachten Sensoren eine Messung der Magnetfeldstärke durchgeführt,
c) auf Grundlage der Messung eine Ausrichtung des Röntgendetektors relativ zum Röntgenstrahler bestimmt.

Die Erfindung hat den Vorteil, die bislang manuell durchgeführte Ausrichtung, die im Sinne der Erfindung durch die Lage des Detektors im Bezug zum Strahler definiert ist, zu automatisieren, bei der der Abstand zwischen Röntgenstrahler und -detektor zum Teil mit einem Maßband ausgemessen wurde. Demgegenüber ermöglicht die Erfindung auch präzisere Lagebestimmungen. Vor allem weil eine Verkippung des Detektors nicht messtechnisch ermittelt, sondern nur per "Augenmaß" korrigiert werden konnte. Die Erfindung vermeidet dabei Fehler bei der Röntgenaufnahme, die dazu führten, dass beispielsweise das Röntgenbild entweder von schlechter Qualität war (Field of View, Unschärfe, Kontrastarmut) oder wiederholt werden musste, was eine zusätzliche Strahlenbelastung bedeutete.

Erfindungsgemäß erfolgt zur Bestimmung die Berechnung einer Translation und/oder Rotation in zumindest je einer Ebene.

Hierdurch können je nach Anzahl der Ebenen und Art des ermittelten Lageversatzes, translatorisch und/oder rotatorisch, diverse mathematische Algorithmen zur akkuraten Schätzung der Lage gezielt ausgewählt bzw. eingesetzt werden.

Erfindungsgemäß werden mindestens zwei Sendemittel zur Aussendung des magnetischen Wechselfeldes zueinander symmetrisch und/oder die mindestens zwei Sensoren zueinander symmetrisch angeordnet betrieben, wobei die Messung derart durchgeführt wird, dass ein Verhältnis der seitens der Sensoren empfangenen Empfangsfeldstärke zueinander gebildet wird und wobei die Bestimmung auf Grundlage des Verhältnisses erfolgt. Damit kann eine einfache Ermittlung/Schätzung der Translation (Translationsversatz) implementiert werden. Die jedoch nur anwendbar ist, soweit keine (Ver-)Kippungs- /Verdrehungsmöglichkeit des Detektors möglich ist. Hiermit wird im Sinne der Erfindung ein reines Gradientenverfahren bereitgestellt.

Um auch bei Verkippung/Verdrehung eine Bestimmung möglich zu machen, kann das Verfahren derart weitergebildet werden, dass mindestens vier Sensoren auf dem Röntgendetektor angeordnet betrieben werden, die Korrelation derart durchgeführt wird, dass durch in Bezug setzen von den zum aktuellen Zeitpunkt empfangenen physikalischen Größen zu einer Referenzgröße eine Positionsschätzung ermittelt wird und die Bestimmung auf Grundlage der Positionsschätzung erfolgt. Hiermit kommt ein im Sinne der Erfindung als Kugelmodell zu bezeichneter Bestimmungsansatz zum Einsatz.

Alternativ, insbesondere falls kein Translationsversatz möglich ist, oder ergänzend, kann das Verfahren gemäß Erfindung derart weitergebildet werden, dass mindestens ein Paar von Sensoren auf dem Röntgendetektor orthogonal zueinander angeordnet betrieben werden, dass die Messung derart durchgeführt wird, dass durch Anwendung trigonometrischer Funktionen, insbesondere des Arkustangens, der Einfallswinkel auf Grundlage der empfangenen physikalischen Größen zwischen den beiden Sensoren ermittelt wird und die Bestimmung auf Grundlage des Einfallswinkels erfolgt. Hierdurch wird eine Rotationsermittlung, d.h. genauer die Bestimmung eines Versatzes der Rotation bzgl. Strahler gewährleistet, die auf Grundlage einer trigonometrischen Funktion, insbesondere dem Arkustangens, erfolgen kann.

Die Genauigkeit der Versatzermittlung der Rotation kann weiter gesteigert werden, wenn die Erfindung derart weitergebildet wird, dass bei einem Einsatz von mehr als einem Paar Sensoren je Paar ein Einfallswinkel bestimmt wird, ein Mittelwert aus dem Einfallswinkel der Paare gebildet wird und die Bestimmung auf Grundlage des Mittelwerts des Einfallswinkels erfolgt.

Bevorzugt wird dabei das Wechselfeld derart erzeugt, dass das Magnetfeld eine Frequenz in einem Bereich zwischen 75 KHz und 150 KHz, insbesondere 125Khz, aufweist. Hierdurch ist ein Frequenzbereich implementiert, der besonders gut geeignet ist, die Materialien und den menschlichen Körper nahezu ohne störende Einflüsse zu durchdringen. Insbesondere 125 KHz sind dabei regulatorisch vorgegeben.

Werden als Sendemittel ein-, zwei- und oder dreidimensionale Spulen betrieben, desto akkurater wird die Bestimmung.

Wird die Erfindung derart weitergebildet, dass als Sensoren Empfangsspulen, 3-Achsen-Beschleunigungssensoren und/oder 3-Achsen-Drehratensensoren betrieben werden, erhält man für die Implementierung diverse Freiheitsgrade, da jede der genannten Sensorarten Eigenschaften mit sich bringt, die für den konkreten Einsatz besonders von Vorteil sein können.

Das erfindungsgemäße Röntgengerät, bestehend aus einem Röntgenstrahler und Röntgendetektor, weist einen Röntgenstrahler auf, der ausgestaltet ist mit Sendern zum Erzeugen und Senden eines magnetischen Wechselfelds sowie einen Röntgendetektor, der ausgestaltet ist mit mindestens zwei zur Erfassung von jeweils zumindest einer mit dem magnetischen Wechselfeld korrelierenden physikalischen Größe, aber zumindest der Magnetfeldstärke, Sensoren und erste Mittel zur Durchführung einer Korrelation der physikalischen Größen. Er weist auch zweite Mittel auf, die derart ausgestaltet sind, dass auf Grundlage der Messung eine Ausrichtung des Röntgendetektors relativ zum Röntgenstrahler bestimmt wird.

Das Röntgengerät stellt eine Implementierung dar, die das Verfahren und damit auch dessen Vorteile verwirklicht. Das erfindungsgemäße Röntgengerät besteht aus einem Röntgenstrahler und Röntgendetektor, mit Mitteln zur Durchführung eines erfindungsgemäßen Verfahrens.

Das Gleiche gilt für die Weiterbildungen des Röntgengeräts, die Mittel zur Durchführung des Verfahrens und seiner Weiterbildungen aufweisen.

Die vorliegende Erfindung wird nun anhand von in den Figuren dargestellten Ausführungsbeispielen der Erfindung näher erläutert. Dabei zeigt

FIG 1     eine den folgenden Ausführungsbeispielen als Ausgangsituation zugrundeliegende Röntgengerätanordnung gemäß Stand der Technik,

FIG 2     schematisch ein erstes Ausführungsbeispiel, welches die erfindungsgemäße Anwendung eines durch die Erfindung definiertes und bezeichnetes Kugelmodell als Messprinzip aufzeigt,

FIG 3     schematisch ein zweites Ausführungsbeispiel, welches die erfindungsgemäße Anwendung eines durch die Erfindung definiertes und bezeichnetes Gradientenmodell als Messprinzip aufzeigt,

FIG 4     ein Ablaufdiagram eines möglichen Ausführungsbeispiels des erfindungsgemäßen Verfahrens.

Die nachfolgend näher geschilderten teilweise nicht dargestellten Ausführungsbeispiele bzw. Ausgestaltungen einzelner Elemente hiervon stellen bevorzugte Ausführungsformen der vorliegenden Erfindung dar. Diese ist jedoch nicht darauf beschränkt.

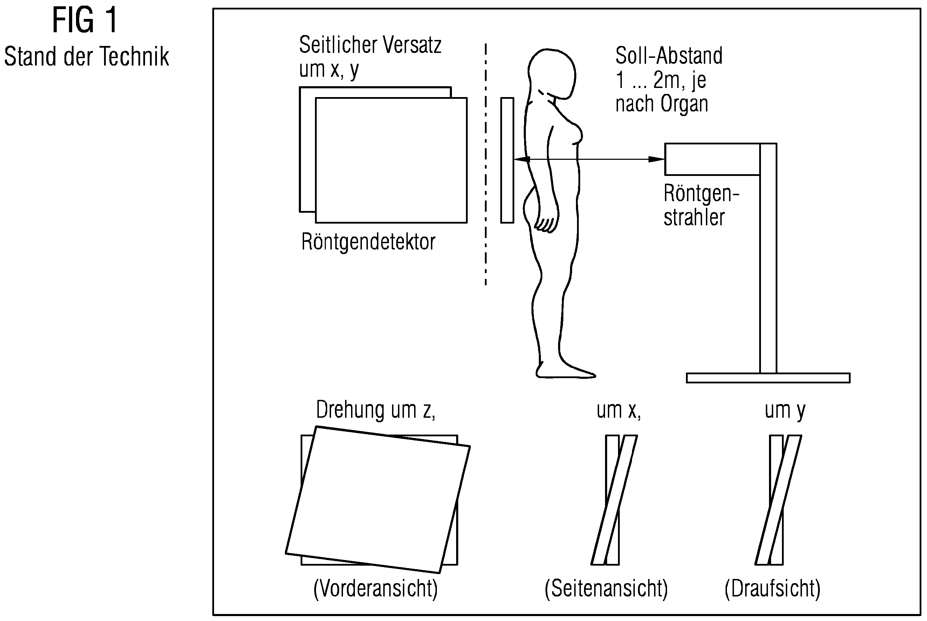

FIG 1 zeigt vereinfacht eine Röntgengeräteanordnung, wie sie aus dem Stand der Technik bekannt ist und die für die folgende Erläuterung der Ausführungsbeispiele als Ausgangssituation betrachtet werden kann.

Zu erkennen ist der prinzipielle Aufbau einer Röntgengeräteanordnung, die aus einem (Röntgen-)Strahler und einem (Röntgen-)Detektor besteht, zwischen denen die zu untersuchende Person platziert wird.

Des Weiteren ist dargestellt, in welcher Weise die Lage des Röntgendetektors sich verändern kann, so dass er zum in der Regel unbeweglichen Strahler einen Versatz aufweist.

Zu erkennen ist, dass eine Verschiebung des Detektors in der x-y-Ebene erfolgen kann. Also ein translatorischer Versatz. Zudem kann der Detektor auch gelegentlich so ausgeführt sein, dass er um eine der drei Achsen im Raum x-y-z gedreht ist.

Die Bestimmung der sich so ergebenden Lage des Detektors wird in der Regel manuell durchgeführt und führt zu den eingangs beschriebenen Nachteilen.

Diese werden erfindungsgemäß dadurch behoben, dass eine automatische Erfassung des Versatzes bereitgestellt wird.

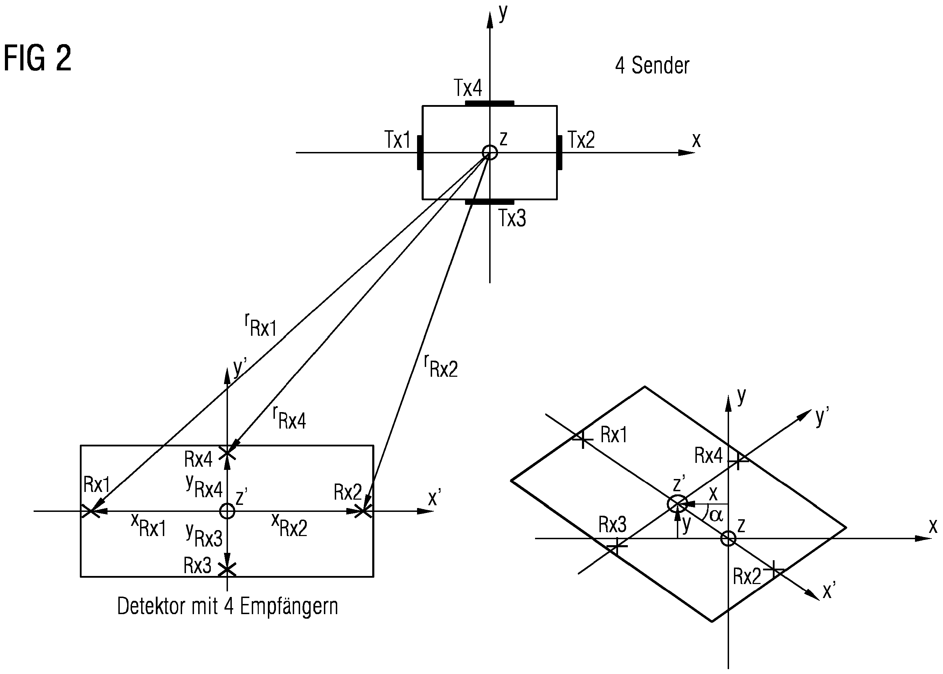

Das in FIG 2 gezeigte Ausführungsbeispiel zeigt die erfindungsgemäße Verwendung von Sendern (mit Strichen am Rand des Strahlers gekennzeichnet) $T_{x1}...T_{x4}$, die auf dem Strahler platziert werden, sowie die Verwendung von Empfängern (mit "x" auf dem Rand des Detektors symbolisch dargestellt) $R_{x1}...R_{x4}$. Diese Platzierung kann wie gezeigt symmetrisch bezüglich der Sender/Empfänger zueinander erfolgen und auch so ausgestaltet sein, dass beispielsweise die Empfänger zueinander orthogonal angebracht werden, wobei alternativ auch mehr als 4 Empfänger $R_{x1}...R_{x4}$ eingesetzt werden können.

Hiervon ausgehend lassen sich für die denkbaren Versatzmöglichkeiten, der Drehwinkel und die Position ermitteln.

Für die Ermittlung des Drehwinkels gelten u.a., wenn gemäß dem Ausführungsbeispiels die Sender ein Wechselmagnetfeld erzeugen und die Empfänger als Spulen ausgestaltet sind, folgende Grundsätze:

- Die Empfangsspannungen werden von mind. zwei orthogonalen Empfangsspulen ermittelt,
- mit Hilfe der trigonometrischen Funktion Arkustangens kann bei Annahme von linear ausgesendeten Magnetfeldern der Einfallswinkel zwischen den beiden Empfangsspulen bestimmt werden,
- bei mehreren Empfangsspulenpaaren wird der Mittelwert des geschätzten Drehwinkels bestimmt,
- bei Einsatz von drei Empfangsspulen lässt sich die Lage allgemein durch trigonometrische Funktionen bestimmen, ist also nicht auf Arkustangens beschränkt,
- die Genauigkeit der Schätzung ist abhängig von:

    o Homogener Feldverteilung erzeugt durch Sendespulen
    o Orthogonalität der Empfangsspulen (Kopplung, Einbau)
    o Signal-zu-Rausch-Abstand.

Ferner gilt grundsätzlich für die erfindungsgemäße Ausführung von Drehwinkeln gemäß Beispiel:

- Eine Verkippung bzw. Drehung im Raum (yaw, pitch, roll) ausschließlich um eine Achse kann über zwei um 90°gekreuzte Empfangsspulen ($R_{x1}$, $R_{x2}$) gemessen werden,
- Einbauort sind die Hauptachsen des Detektors

    o yaw □ Empfangsspulen liegen in der x'y'-Ebene,
    o pitch □ Empfangsspulen liegen in der x'z'-Ebene,
    o roll □ Empfangsspulen liegen in der y'z'-Ebene,

- geschätzter Winkel = atan (Messung an $R_{x1}$/Messung an $R_{x2}$)
- um eine Verkippung um zwei Achsen zu erfassen ist als eine Weiterbildung eine 3D-Empfangsspule notwendig,
- Kalibration der Empfangsspulen erfolgt vor einer Winkelschätzung,
- zu beachten ist, dass eine Winkelmessung mittels trigonometrischer Funktion nur bei einer homogenen Phase im Raum anwendbar ist.

Für die Ermittlung der Position des Detektors bei der gegebenen Ausgangssituation sollen zwei Ausführungsbeispiele im Wesentlichen gekennzeichnet durch den Einsatz von Messprinzipien zum Einsatz kommen, eines, das als Kugelmodell bezeichnet werden kann und eines, welches als Gradientenmodell/- verfahren bezeichnet werden kann, wobei das Kugelmodell gemäß Ausführungsbeispiel sich dadurch auszeichnet, dass

- die Position des Detektors relativ zum Sender mittels eines Kugelmodells geschätzt wird,
- die gezeigten 4 Empfänger zur Schätzung der Position x,y,z des Detektors zum Einsatz kommen,
- das Kugelmodell dabei auf einem empirischen Modell basiert, wobei
- das empirische Modell auf Messungen der Feldstärke, die einmalig bei relevanten Abständen zwischen Sender und Detektor und in einem relevanten Bereich bei konstantem Abstand ermittelt werden, basiert, und wobei
- für jeden gemessenen Wert eine Kostenfunktion für jeden Empfänger minimiert wird.

Beim Kugelmodell kommen dabei folgende mathematische, die Gegebenheiten der Röntgenanordnung beschreibende/modellierende, Gleichungen zum Einsatz:

$$r_{Rx1} = \sqrt{(x + x_1)^2 + (y + y_1)^2 + (z + z_1)^2}$$

$$r_{Rx2} = \sqrt{(x + x_2)^2 + (y + y_2)^2 + (z + z_2)^2}$$

$$r_{Rx3} = \sqrt{(x + x_3)^2 + (y + y_3)^2 + (z + z_3)^2}$$

$$r_{Rx4} = \sqrt{(x + x_4)^2 + (y + y_4)^2 + (z + z_4)^2}$$

Hiermit sind die Modellgleichungen für die 4 Empfänger gegeben. Dabei ist für einen Empfänger mit Offset zum Mittelpunkt des Detektors und mit $x_n$, $y_n$, $z_n$, abhängig von den Raumwinkeln yaw $\alpha$, roll $\beta$, und pitch $\gamma$ grundsätzlich die Eulermatrix M mit

$$\begin{pmatrix} x_n \\ y_n \\ z_n \end{pmatrix} = M \cdot \begin{pmatrix} x_{Rxn} \\ y_{Rxn} \\ z_{Rxn} \end{pmatrix}$$

gegeben. Bei einer Rotation um die z-Achse wie in der Figur dargestellt (mit einem Wert des Winkels $\alpha$=45°) würde sich die Matrix wie folgt ergeben:

$$M = \begin{pmatrix} \cos\alpha & -\sin\alpha & 0 \\ \sin\alpha & \cos\alpha & 0 \\ 0 & 0 & 1 \end{pmatrix}$$

woraus der Drehwinkel bestimmt werden kann.

Die Position, d.h. die Verschiebung gemäß Darstellung um x=y= 10 cm, des Detektors zum Mittelpunkt bezüglich aller Sender $T_{xn}$ wird dabei aus den Empfangsspannungen aller Empfänger $R_{xn}$ geschätzt.

Dabei gilt für die Schätzung der x-y Translation mittels Kugelgleichung, dass

- die xy-Schätzung auf einem Polynom basiert, wobei für das Polynom gilt, dass

  o die Feldverteilung nicht symmetrisch ist,
  o ein Polynom den Mittelwert aller Kurven bestimmt,
  o mit Hilfe des Polynoms jeder gemessenen Empfangsspannung ein Radius zugeordnet wird,

- für die Schätzung genügen erfindungsgemäß also mindestens drei Empfangsspulen (ausreichend da bzw. wenn der z-Abstand konstant und bekannt ist),
- ein Schätzfehler sich mit Hinzunahme weiterer Empfänger minimiert,
- es geeignet ist für Verschiebungen geringeren Ausmaßes, da mit größer werdenden Verschiebungen Messfehler zunehmen, soweit Polynome zur Schätzung verwendet werden,
- Schätzprinzip auch bei einer Verdrehung des Detektors anwendbar ist,
- der mittlere euklidische Abstand 1,7 cm beträgt.

Die durch eindimensionale Empfängerspulen erreichte Genauigkeit ist ausreichend und bietet eine günstige Lösung. Genauere Ergebnisse lassen sich auch durch den Einsatz von zweidimensionalen oder dreidimensionalen Spulen erzielen.

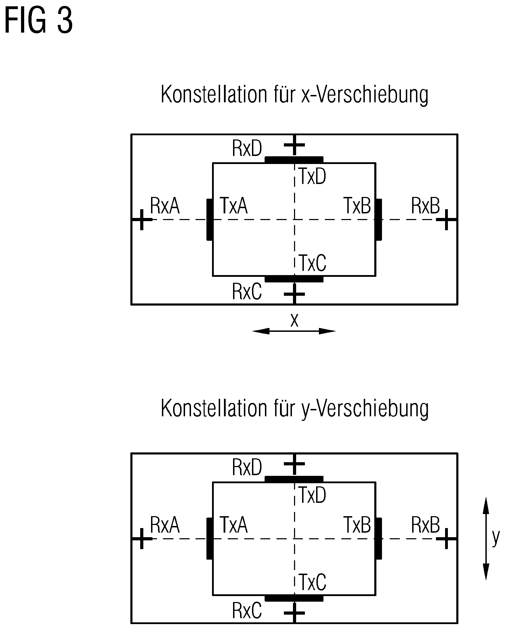

Wohingegen das Gradientenverfahren gemäß Ausführungsbeispiel, das auf Grundlage einer Ausgangsituation wie in FIG 3 angedeutet - d.h. rein translatorische Verschiebung - sich dadurch auszeichnet, dass

- die xy-Verschiebung mittels Gradient bestimmt wird,
- die Verschiebung über ein Empfangsverhältnis einer symmetrischen Sende- und Empfangskonstellation bestimmt werden kann, wobei
- dies nur anwendbar ist, wenn keine Drehung/Verkippung vorliegt,
- der Gradient nur für einen Abstand gültig ist, so dass das Prinzip eingesetzt wird, dass im Fall, dass bei zwei sich gegenüberliegenden Empfängern nicht die gleiche Empfangsfeldstärke aufgewiesen wird, eine Verschiebung vorliegt,
- der mittlere euklidische Abstand mit 0,3 cm gegeben ist.

Es gilt ferner, dass ein geeignetes Polynom für die Schätzung gemäß Ausführungsbeispiel der Erfindung unter Beachtung folgender Punkte bestimmt werden kann:

- Die gemessenen Empfangsspannungen hängen von der Position der Empfangsspulen auf dem Detektor ab ($R_{x1}$ und $R_{x2}$ haben zur Detektormitte einen größeren Abstand als $R_{x3}$ und $R_{x4}$).
- Für eine Grobschätzung des Abstandes genügt die Bestimmung eines Polynoms, welches auf der Addition der Spannungen aller Empfangsspulen basiert.
- Für eine genaue Abstandsschätzung ist ein Polynom zu bestimmen, dass sich aus den Spannungen von Empfangsspulen ergibt, die einen identischen Abstand zur Mitte des Detektors aufweisen.

Hierdurch kann, sofern jeder Empfangsspule abhängig von ihrer Position auf der Detektorplatte das entsprechende Polynom zugeordnet wird, eine gute Korrelation von Radien zu Empfangsspannungen bei verschiedenen Abständen gewährleistet werden.

Da gemäß einer alternativen bzw. ergänzenden Weiterbildung mittels Polynom auch für jeden Empfänger eine Kugelgleichung aufgestellt werden kann, kann auch eine Schätzung mittels Kugelmodell erfolgen, wobei die z-Komponente sehr gut geschätzt werden kann, wenn die geschätzte x- und y-Komponente idealerweise immer 0 entsprechen.

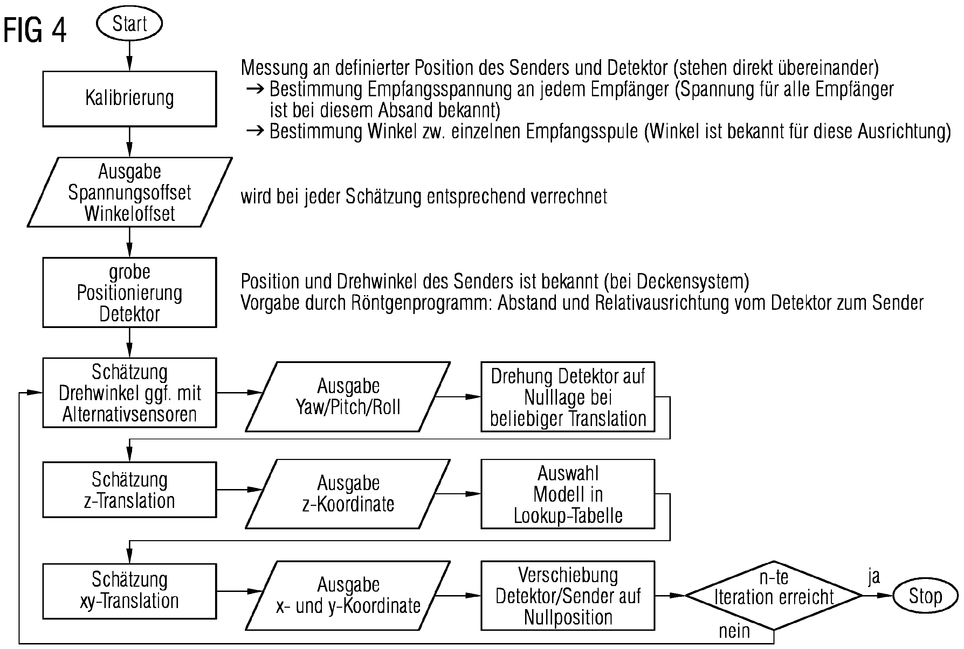

Implementiert man nun diese Messprinzipien bzw. Modelle als erfindungsgemäßes Verfahren, kann ein Ausführungsbeispiel gemäß FIG 4 zum Einsatz kommen.

Die FIG 4 zeigt schematisch ein Ablaufdiagramm des Ausführungsbeispiels und beginnt in einem ersten Schritt S1 bei einem Ausgangszustand "Start" von dem aus in einem zweiten Schritt S2 eine Kalibrierung erfolgt, die in einem dritten Schritt S3 als Ergebnis einen Spannungs- und Winkeloffset ermittelt, in dem eine Messung an einer definierten Position des Senders und des Detektors erfolgt, beispielweise, dass diese direkt übereinander stehen, wobei eine Empfangsspannung an jedem Empfänger sowie Winkel zwischen den einzelnen Empfangsspulen bestimmt wird.

In einem vierten Schritt S4 erfolgt dann eine grobe Positionierung, die eine anschließende Schätzung des Drehwinkels, beispielsweise gemäß einer der oben beschriebenen Ansätze oder einer Kombination hiervon, in einem fünften Schritt S5 nachfolgt.

Hierauf folgt in einem sechsten Schritt S6 die Ausgabe der Raumwinkel Yaw/Pitch/Roll, der eine Drehung des Detektors auf Nulllage (bei beliebiger Translation) in einem siebten Schritt S7 folgt.

Dies führt zur Schätzung der z-Translation in einem achten Schritt S8 und Ausgabe der z-Koordinate in einem neunten Schritt S9. Im Anschluss wird dann in einem zehnten Schritt S10 ein Modell, beispielsweise der oben erläuterten, ausgewählt, mit dem in einem elften Schritt S11 die xy-Translation geschätzt und in einem zwölften Schritt S12 eine Verschiebung des Detektors/Senders auf Nullposition durchgeführt werden kann.

Durch eine Auswertung in einem dreizehnten Schritt S13, ob n-Iterationen erreicht worden sind, wird im positiven Fall das Verfahren beendet und geht in einem vierzehnten Schritt S14 in den Zustand "Stop" über.

Falls nicht, werden die Schritte fünf bis elf S5...S11 wiederholt.

Der in diesem Dokument beschriebene Ansatz ist nicht auf die in den Figuren erläuterten Beispiele beschränkt, sondern umfasst alle durch die Ansprüche umfassten Lösungen, die ausschließlich auf Amplitudenmessungen und deren Auswertung basieren und bei denen die Lage im Raum von Objekten und dessen Position mit Hilfe des Einsatzes von einer hinsichtlich des Typs homogenen Auswahl von Sensoren aus einer grundsätzlich möglichen Auswahl aus mehreren Sensortypen (Beschleunigungssensor, Gyro,) bewerkstelligt werden, die der fachfremden Lageregelung von Flugobjekten vergleichbar eingesetzt werden. Vergleichbar, da deren Anwendung im medizinischen Umfeld und im Innenbereich von Gebäuden nicht vorgesehen und daher zum Teil nur mit Anpassungen/Einschränkungen möglich ist.

**Patentansprüche**

1. Verfahren zum Betreiben eines aus einem Röntgenstrahler und Röntgendetektor bestehenden Röntgengerätes, derart ausgestaltet, dass

   - am Röntgenstrahler ein magnetisches Wechselfeld erzeugt und ausgesandt wird,
   - durch mindestens zwei am Röntgendetektor angebrachten Sensoren jeweils zumindest eine mit dem magnetischen Wechselfeld korrelierenden Magnetfeldstärke gemessen wird,
   - auf Grundlage der Messung eine Ausrichtung des Röntgendetektors relativ zum Röntgenstrahler bestimmt wird, wobei zur Bestimmung die Berechnung einer Translation und/oder Rotation in zumindest je einer Ebene erfolgt,
   - mindestens zwei Sendemittel zur Aussendung des magnetischen Wechselfeldes zueinander symmetrisch und/oder die mindestens zwei Sensoren zueinander symmetrisch angeordnet betrieben werden,
   - die Messung derart durchgeführt wird, dass ein Verhältnis der seitens der Sensoren empfangenen Empfangsfeldstärke zueinander gebildet wird,
   - die Bestimmung auf Grundlage des Verhältnisses erfolgt.

2. Verfahren nach Anspruch 1, **dadurch gekennzeichnet, dass**

   - mindestens vier Sensoren auf dem Röntgendetektor angeordnet betrieben werden,
   - die Messung derart durchgeführt wird, das durch in Bezug setzen von den zum aktuellen Zeitpunkt empfangenen physikalischen Größen zu einer Referenzgröße eine Positionsschätzung ermittelt wird,
   - die Bestimmung auf Grundlage der Positionsschätzung erfolgt.

3. Verfahren nach Anspruch 1 oder 2, **dadurch gekennzeichnet, dass**

   - mindestens ein Paar von Sensoren auf dem Röntgendetektor orthogonal zueinander angeordnet betrieben werden,
   - die Messung derart durchgeführt wird, das durch Anwendung trigonometrischer Funktionen der Einfallswinkel auf Grundlage der empfangenen physikalischen Größen zwischen den beiden Sensoren ermittelt wird,
   - die Bestimmung auf Grundlage des Einfallswinkels erfolgt.

4. Verfahren nach Anspruch 3, **dadurch gekennzeichnet, dass** bei einem Einsatz von mehr als einem Paar Sensoren

   - je Paar ein Einfallswinkel bestimmt wird,
   - ein Mittelwert aus den Einfallswinkel der Paare gebildet wird,
   - die Bestimmung auf Grundlage des Mittelwerts des Einfallswinkels erfolgt.

5. Verfahren nach einem der vorhergehenden Ansprüche, **dadurch gekennzeichnet, dass** das Wechselfeld derart erzeugt wird, dass das Magnetfeld eine Frequenz in einem Bereich zwischen 75 KHz und 150 KHz aufweist.

6. Verfahren nach einem der vorhergehenden Ansprüche, **dadurch gekennzeichnet, dass** als Sendemittel ein-, zwei- und oder dreidimensionale Spulen betrieben werden.

7. Verfahren nach einem der vorhergehenden Ansprüche, **dadurch gekennzeichnet, dass** als Sensoren Empfangsspulen, 3-Achsen-Beschleunigungssensoren und/oder 3-Achsen-Drehratensensoren betrieben werden.

8. Röntgengerät bestehend aus einem Röntgenstrahler und Röntgendetektor, umfassend:

   - den Röntgenstrahler, der mit mindestens zwei Sendemitteln zum Erzeugen und Senden eines magnetischen Wechselfelds ausgestaltet ist,
   - den Röntgendetektor, der mit mindestens zwei Sensoren zur Erfassung von jeweils zumindest einer mit dem magnetischen Wechselfeld korrelierenden Magnetfeldstärke ausgestaltet ist, wobei die mindestens zwei Sendemittel zur Aussendung des magnetischen Wechselfeldes zueinander symmetrisch und/oder die mindestens zwei Sensoren zueinander symmetrisch angeordnet sind,
   - Mittel zur Bildung des Verhältnisses der seitens der Sensoren empfangenen Empfangsfeldstärke,
   - Mittel zur Bestimmung einer Ausrichtung des Röntgendetektors relativ zum Röntgenstrahler, wobei zur Bestimmung die Berechnung einer Translation und/oder Rotation in zumindest je einer Ebene erfolgt und wobei die Bestimmung auf Grundlage des Verhältnisses erfolgt.

**Claims**

1. Method for operating an x-ray device consisting of an x-ray emitter and an x-ray detector, configured in such a way that

   - an alternating magnetic field is produced and emitted at the x-ray emitter,
   - at least two sensors attached on the x-ray detector are each used to measure at least one magnetic field strength correlating with the alternating magnetic field,
   - an alignment of the x-ray detector relative to the x-ray emitter is determined on the basis of the measurement, wherein a translation and/or rotation is calculated at least in a plane in each case for the determination,
   - at least two transmission means for emitting the alternating magnetic field are operated in a manner arranged symmetrically with respect to one another and/or the at least two sensors are operated in a manner arranged symmetrically with respect to one another,
   - the measurement is carried out in such a way that a ratio is formed of the reception field strength as received on the part of the sensors,
   - the determination is carried out on the basis of the ratio.

2. Method according to Claim 1, **characterized in that**

   - at least four sensors are operated in a manner arranged on the x-ray detector,
   - the measurement is carried out in such a way that a position estimate is ascertained by relating physical variables received at the current time to a reference variable,
   - the determination is carried out on the basis of the position estimate.

3. Method according to Claim 1 or 2, **characterized in that**

   - at least one pair of sensors are operated in a manner arranged orthogonal with respect to one another on the x-ray detector,
   - the measurement is carried out in such a way that the angle of incidence is ascertained on the basis of the received physical variables between the two sensors by applying trigonometric functions,
   - the determination is carried out on the basis of the angle of incidence.

4. Method according to Claim 3, **characterized in that**, if more than one pair of sensors is used,

   - an angle of incidence is determined for each pair,
   - a mean value is formed from the angles of incidence of the pairs,
   - the determination is carried out on the basis of the mean value of the angle of incidence.

5. Method according to one of the preceding claims, **characterized in that** the alternating field is produced in such a way that the magnetic field has a frequency in a range between 75 kHz and 150 kHz.

6. Method according to one of the preceding claims, **characterized in that** one-dimensional, two-dimensional and/or three-dimensional coils are operated as transmission means.

7. Method according to one of the preceding claims, **characterized in that** reception coils, 3-axis acceleration sensors and/or 3-axis rotational speed sensors are operated as sensors.

8. X-ray device consisting of an x-ray emitter and an x-ray detector, comprising:

   - the x-ray emitter, which is configured with at least two transmission means for producing and transmitting an alternating magnetic field,
   - the x-ray detector, which is configured with at least two sensors for respectively capturing at least one magnetic field strength correlating with the alternating magnetic field, wherein the at least two transmission means for emitting the alternating magnetic field are operated in a manner arranged symmetrically with respect to one another and/or the at least two sensors are operated in a manner arranged symmetrically with respect to one another,

- means for forming the ratio of the reception field strength as received on the part of the sensors,
- means for determining an alignment of the x-ray detector relative to the x-ray emitter, wherein a translation and/or rotation is calculated at least in a plane in each case for the determination and wherein the determination is carried out on the basis of the ratio.

**Revendications**

1.  Procédé pour faire fonctionner un appareil à rayons X, constitué d'un émetteur de rayons X et d'un détecteur de rayons X, tel que

    - on produit et on envoie à l'émetteur de rayons X un champ magnétique alternatif,
    - par au moins deux capteurs montés sur le détecteur de rayons X, on mesure respectivement au moins une intensité du champ magnétique en corrélation avec le champ magnétique alternatif,
    - sur la base de la mesure, on détermine une orientation du détecteur de rayons X par rapport à l'émetteur de rayons X, dans lequel pour la détermination on effectue le calcul d'une translation et/ou d'une rotation dans au moins respectivement un plan,
    - on fait fonctionner deux moyens d'émission pour l'émission du champ magnétique alternatif symétriquement l'un par rapport à l'autre et/ou on dispose les au moins deux capteurs symétriquement l'un par rapport à l'autre,
    - on effectue la mesure de manière à former un rapport des intensités de champ de réception reçues par les capteurs, et
    - la détermination a lieu sur la base du rapport.

2.  Procédé suivant la revendication 1, **caractérisé en ce que**

    - on fait fonctionner au moins quatre capteurs montés sur le détecteur de rayons X,
    - on effectue la mesure de manière à déterminer une estimation de position par la mise en rapport des grandeurs physiques reçues à l'instant en cours à une grandeur de référence,
    - la détermination a lieu sur la base de l'estimation de position.

3.  Procédé suivant la revendication 1 ou 2, **caractérisé en ce que**

    - on fait fonctionner une paire de capteurs en les disposant orthogonalement l'un par rapport à l'autre sur le détecteur de rayons X,
    - on effectue la mesure de manière à déterminer entre les deux capteurs, par application de fonctions trigonométriques, l'angle d'incidence sur la base des grandeurs physiques reçues,
    - la détermination a lieu sur la base de l'angle d'incidence.

4.  Procédé suivant la revendication 3, **caractérisé en ce que** si l'on utilise plus d'une paire de capteurs,

    - on détermine pour chaque paire un angle d'incidence,
    - on forme une valeur moyenne à partir des angles d'incidence des paires,
    - la détermination a lieu sur la base de la valeur moyenne de l'angle d'incidence.

5.  Procédé suivant l'une des revendications précédentes, **caractérisé en ce qu'**on produit le champ alternatif de manière à ce que le champ magnétique ait une fréquence dans une plage comprise entre 75 KHz et 150 KHz.

6.  Procédé suivant l'une des revendications précédentes, **caractérisé en ce que** l'on fait fonctionner comme moyen d'émission une bobine à une dimension, à deux dimensions ou à trois dimensions.

7.  Procédé suivant l'une des revendications précédentes, **caractérisé en ce que** l'on fait fonctionner comme capteurs des bobines de réception, des capteurs d'accélération à trois axes et/ou des capteurs de vitesse angulaire à trois axes.

8.  Appareil à rayons X constitué d'un émetteur de rayons X et d'un détecteur de rayons X comprenant :

    - l'émetteur de rayons X, qui est conformé en ayant au moins deux moyens d'émission pour la production et l'émission d'un champ magnétique alternatif,
    - le détecteur de rayons X, qui est conformé en ayant au moins deux capteurs de détection de respectivement au moins une intensité du champ magnétique en corrélation avec le champ magnétique alternatif, dans lequel les au moins deux moyens d'émission sont disposés pour l'émission du champ magnétique alternatif en étant symétrique l'un de l'autre et/ou les au moins deux capteurs sont disposés en étant symétriques l'un de l'autre,
- un moyen de formation du rapport des intensités du champ de réception reçues par les capteurs,
- un moyen de détermination d'une orientation du détecteur de rayons X par rapport à l'émetteur de rayons X, dans lequel pour la détermination on effectue le calcul d'une translation et/ou d'une rotation dans au moins respectivement un plan, dans lequel la détermination a lieu sur la base du rapport.

# FIG 1
## Stand der Technik

Seitlicher Versatz
um x, y

Soll-Abstand
1 ... 2m, je
nach Organ

Röntgendetektor

Röntgenstrahler

Drehung um z,

um x,

um y

(Vorderansicht)

(Seitenansicht)

(Draufsicht)

EP 3 239 700 B1

FIG 2

4 Sender

Tx4

Tx1   z   Tx2

Tx3

$r_{Rx1}$

$r_{Rx4}$

$r_{Rx2}$

Rx1

Rx4
$y_{Rx4}$   z'   Rx2
$x_{Rx1}$   $y_{Rx3}$   $x_{Rx2}$

Rx3

Detektor mit 4 Empfängern

Rx1

Rx4

z'   x
$\alpha$
Rx3   y   z

Rx2

EP 3 239 700 B1

# FIG 3

Konstellation für x-Verschiebung

Konstellation für y-Verschiebung

**FIG 4**

```
        ( Start )
           │
           ▼
   ┌──────────────┐
   │ Kalibrierung │      Messung an definierter Position des Senders und Detektor (stehen direkt übereinander)
   └──────────────┘      → Bestimmung Empfangsspannung an jedem Empfänger (Spannung für alle Empfänger
           │                 ist bei diesem Absand bekannt)
           │             → Bestimmung Winkel zw. einzelnen Empfangsspule (Winkel ist bekannt für diese Ausrichtung)
           ▼
   ╱──────────────╱
  ╱   Ausgabe    ╱
 ╱ Spannungsoffset╱       wird bei jeder Schätzung entsprechend verrechnet
╱   Winkeloffset ╱
╱──────────────╱
           │
           ▼
   ┌──────────────┐
   │    grobe     │       Position und Drehwinkel des Senders ist bekannt (bei Deckensystem)
   │ Positionierung│      Vorgabe durch Röntgenprogramm: Abstand und Relativausrichtung vom Detektor zum Sender
   │   Detektor   │
   └──────────────┘
           │
           ▼
   ┌──────────────┐        ╱──────────────╱      ┌──────────────────┐
   │  Schätzung   │       ╱    Ausgabe   ╱       │ Drehung Detektor auf│
   │Drehwinkel ggf. mit│→ ╱  Yaw/Pitch/Roll ╱ → │   Nulllage bei   │
   │Alternativsensoren│  ╱──────────────╱        │ beliebiger Translation│
   └──────────────┘                             └──────────────────┘
           │                                              │
           ▼                                              ▼
   ┌──────────────┐        ╱──────────────╱      ┌──────────────────┐
   │  Schätzung   │       ╱    Ausgabe   ╱       │     Auswahl      │
   │ z-Translation│  →    ╱  z-Koordinate ╱  →   │    Modell in     │
   └──────────────┘      ╱──────────────╱        │  Lookup-Tabelle  │
           │                                     └──────────────────┘
           ▼                                              │
   ┌──────────────┐        ╱──────────────╱      ┌──────────────────┐      ◇ n-te          ja
   │  Schätzung   │       ╱    Ausgabe   ╱       │   Verschiebung   │    ◇ Iteration erreicht ◇ → ( Stop )
   │ xy-Translation│ →    ╱x- und y-Koordinate╱→│ Detektor/Sender auf│   ◇                ◇
   └──────────────┘      ╱──────────────╱        │   Nullposition   │      nein
                                                 └──────────────────┘
```

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2012127117 A1